# EUROPEAN PATENT SPECIFICATION

(11) **EP 3 211 004 B1**
(45) Date of publication and mention of the grant of the patent: **06.12.2023**
(21) Application number: 15852326.6
(22) Date of filing: 23.10.2015
(51) Int. Cl.: C07K 16/24, A61P 3/10, A61P 9/00, A61K 39/00

(54) **USE OF MACROPHAGE INFLAMMATORY PROTEIN-1 (MIP-1 ) INHIBITOR FOR IMPROVING TISSUE ISCHEMIA AND DIABETES VASCULOPATHY BY PROMOTING ANGIOGENESIS**
VERWENDUNG EINES MACROPHAGENENTZÜNDUNGSPROTEIN-1(MIP-1)-INHIBITORS ZUR VERBESSERUNG VON GEWEBEISCHÄMIE UND DIABETISCHER VASKULOPATHIE DURCH FÖRDERUNG DER ANGIOGENESE
UTILISATION D'INHIBITEUR DE LA PROTÉINE INFLAMMATOIRE DES MACROPHAGES 1 (MIP-1 ) POUR AMÉLIORER L'ISCHÉMIE TISSULAIRE ET LA VASCULOPATHIE DIABÉTIQUE EN FAVORISANT L'ANGIOGENÈSE

(30) Priority: 24.10.2014 US 201462068475 P
(43) Date of publication of application: 30.08.2017
(73) Proprietor: National Yang-Ming University, Taipei 112 , Taiwan (TW); Taipei Veterans General Hospital, Taipei City, Taiwan 11217 (TW)
(72) Inventor: CHEN, Jaw-Wen, Taipei City (TW); CHANG, Ting-Ting, Taoyuan City Taoyuan County (TW)
(74) Representative: Viering, Jentschura & Partner mbB Patent- und Rechtsanwälte
(86) International application number: PCT/CN2015/092766
(87) International publication number: WO 2016/062280

(56) References cited:
- WO-A1-2011/025962
- CN-A- 102 782 148
- CAMERON M J ET AL: "Differential expression of CC chemokines and the CCR5 receptor in the pancreas is associated with progression to type I diabetes", THE JOURNAL OF IMMUNOLOGY, THE AMERICAN ASSOCIATION OF IMMUNOLOGISTS, US, vol. 165, no. 2, 15 July 2000 (2000-07-15) , pages 1102-1110, XP002278898, ISSN: 0022-1767
- SUNILK KOTA ET AL: "Aberrant angiogenesis: The gateway to diabetic complications", INDIAN JOURNAL OF ENDOCRINOLOGY AND METABOLISM, vol. 16, no. 6, 1 January 2012 (2012-01-01), page 918, XP055466107, ISSN: 2230-8210, DOI: 10.4103/2230-8210.102992
- C. MEAGHER ET AL: "Neutralization of Interleukin-16 Protects Nonobese Diabetic Mice From Autoimmune Type 1 Diabetes by a CCL4-Dependent Mechanism", DIABETES, vol. 59, no. 11, 6 August 2010 (2010-08-06), pages 2862-2871, XP055466376, US ISSN: 0012-1797, DOI: 10.2337/db09-0131
- WEI CHEN ET AL: "Insulin-Like Growth Factor (IGF)-I/IGF-Binding Protein-3 Complex: Therapeutic Efficacy and Mechanism of Protection against Type 1 Diabetes", ENDOCRINOLOGY, vol. 145, no. 2, 1 February 2004 (2004-02-01), pages 627-638, XP055466392, US ISSN: 0013-7227, DOI: 10.1210/en.2003-1274
- C. MEAGHER ET AL: "CCL4 Protects From Type 1 Diabetes by Altering Islet -Cell-Targeted Inflammatory Responses", DIABETES, vol. 56, no. 3, 27 February 2007 (2007-02-27), pages 809-817, XP055466457, US ISSN: 0012-1797, DOI: 10.2337/db06-0619
- ANNA RYDÉN ET AL: "Altered immune profile from pre-diabetes to manifestation of type 1 diabetes", DIABETES RESEARCH AND CLINICAL PRACTICE, vol. 100, no. 1, 1 April 2013 (2013-04-01) , pages 74-84, XP055465986, NL ISSN: 0168-8227, DOI: 10.1016/j.diabres.2013.01.014
- TING-TING CHANG ET AL: "Emerging role of chemokine CC motif ligand 4 related mechanisms in diabetes mellitus and cardiovascular disease: friends or foes?", CARDIOVASCULAR DIABETOLOGY, vol. 15, no. 1, 24 August 2016 (2016-08-24), XP055466061, DOI: 10.1186/s12933-016-0439-9
- Brian L. Furman: "Streptozotocin-Induced Diabetic Models in Mice and Rats : Streptozotocin-Induced Diabetic Models" In: "Current Protocols in Pharmacology", 1 September 2015 (2015-09-01), John Wiley & Sons, Inc., Hoboken, NJ, USA, XP055466332, ISBN: 978-0-471-14175-4 pages 5.47.1-5.47.20, DOI: 10.1002/0471141755.ph0547s70,
- TING-TING CHANG ET AL: "Direct Renin Inhibition with Aliskiren Improves Ischemia-Induced Neovasculogenesis in Diabetic Animals via the SDF-1 Related Mechanism", PLOS ONE, vol. 10, no. 8, 25 August 2015 (2015-08-25), page e0136627, XP055466563, DOI: 10.1371/journal.pone.0136627
- M. C. YODER: "Human Endothelial Progenitor Cells", COLD SPRING HARBOR PERSPECTIVES IN MEDICINE, vol. 2, no. 7, 28 September 2011 (2011-09-28), pages a006692-a006692, XP055466600, DOI: 10.1101/cshperspect.a006692
- TATARA, Y. ET AL.: 'Macrophage Inflammatory Protein-1beta Induced Cell Adhesion with Increased Intracellular Reactive Oxygen Species' JOURNAL OF MOLECULAR AND CELLULAR CARDIOLOGY. vol. 47, 26 March 2009, pages 104 - 111, XP026159849
- DOBACZEWSKI, M. ET AL.: 'CCRS Signaling Suppresses Inflammation and Reduces Adverse Remodeling of the Infarcted Heart, Mediating Recruitment of Regulatory T Cells' THE AMERICAN JOURNAL OF PATHOLOGY. vol. 176, no. 5, 31 May 2010, pages 2177 - 2186, XP055274812
- LUO, XIAOLING ET AL.: 'Antitumor Effects of MIP-10 Genes Mediated by Tumor Body Injection Adenovirus and Mechanism Analysis Thereof' THE 7TH NATIONAL ACADEMIC CONFERENCE ON TUMOR BIOTHERAPY. 18 October 2001, page 109, XP009502618

## Description

### BACKGROUND OF THE INVENTION

### Technical Field of the Invention

The present invention relates to a macrophage inflammatory protein-1β (MIP-1β) inhibitor for use in treating tissue ischemia in a diabetic subject, wherein the MIP-1 beta inhibitor is an antibody against MIP-1 beta.

### Background

Vascular complications are the major cause of morbidity and early mortality of diabetic patients, especially in type 2 DM. It is also a common case that diabetic patients are amputated due to the peripheral vascular lesions. There are close links between diabetic vascular diseases and general risk factors of atherosclerosis such as hypertension, high blood lipid and hyperglycemia. These cardiovascular diseases have been increasingly recognized to be vascular inflammation related diseases. However, the treatment efficiency on the cardiovascular disease in diabetic patients is worse than that on the general arteriosclerosis, at present. One of the main reasons is the lack of effective treatment strategy to control the inflammatory responses in diabetic vascular diseases.

Currently, the prevention and treatment effects of vascular disease drugs are mainly focused on the mechanisum of controlling general risk factors of atherosclerosis such as blood pressure, blood lipids and blood sugar. These drugs could only delay the onset of diabetes-related vascular disease, or control the disorders after the onset of diabetes-related vascular lesions, which has not been able to control the vascular inflammation of diabetes. Thus, there is a need to seek a new treatment for diabetic vasculopathy by directly regulating the of vascular inflammation diabetes, protecting the blood vessels, enhancing angiogenesis and improving tissue ischemia.

There are certain articles showed that endothelial progenitor cells (EPCs) are essential in neovasculargenesis to maintain the functional and integrity of the vascular endothelial layer. In ischemic tissue, EPCs are also shown to have effects of promoting angiogenesis. The new blood vessels could provide the blood circulation needed in hypoxic tissue, and to reduce the organ damages caused by the lack of oxygen (Asahara, T., et al. EMBO J 18, 3964-3972 ,1999; Gallagher, K.A., et al. J Clin Invest 117, 1249-1259 ,2007). Therefore, it is also an important subject to find out the way of improving arteriosclerosis and increasing the angiogenesis in hypoxic tissue by promoting the endothelial progenitor cells.

EPC mobilization from the bone marrow compartment is mediated by the SDF-1 receptor CXCR4. And, blockade of CXCR4 on circulating cells prevents progenitor cell recruitment to ischemic sites (Ceradini, D.J., et al. Nat Med 10, 858-864 ,2004). In diabetic mice, expression of VEGF and SDF-1 in wound tissue, the number of EPC in peripheral blood, and the expression level of CXCR4 on EPC are decreased (Ceradini, D.J., et al. Journal of Biological Chemistry 283, 10930-10938 ,2008).

Macrophage inflammatory protein-1β (MIP-1β, also known as CCL4) is a member of the CC chemokine family that is first isolated from culture medium of LPS-activated macrophage. MIP-1β has a molecular weight (MW) of 7.8 kDa. The protein structure of MIP-1β is constituted as a precursor of 92 amino acids. Mature secreted proteins as 69 amino acids are generated by peptidases that cleave hydrophobic signal peptides. The upregulation of MIP-1β was observed in DM and cardiovascular diseases. *In vitro,* MIP-1β (also known as CCL4) was able to induce reactive oxygen species production and adhesion of THP-1 cells to human umbilical vein endothelial cells. CCL4 directly induced cell adhesion to endothelial cells through oxidative stress via PI3k-Rac1 cascades (Tatara Y et al., J Mol Cell Cardiol 47:104-111, 2009). Also, CCL4 secreted from macrophages under high glucose condition is capable to induce the endothelial expression of adhesion molecule such as E-selectin in the *in vitro* study (Chen TC, et al., J Biol Chem 286:25564-25573, 2011).

In the animal model of myocardial infarction, chemokine induction in the infarct heart mediates recruitment of leukocyte subsets with distinct properties. CCL4 and its receptor CCR5 were significantly induced in the infarct mouse myocardium (Dobaczewski M, et al, Am J Pathol 176:2177-2187, 2010). However, the *in vivo* evidence indicating the direct contribution of CCL4 to vascular and myocardial injury is still lacking.

Cameron et al. (Differential expression of CC chemokines and the CCR5 receptor in the pancreas is associated with progression to type I diabetes, J. Immunol. 2002 Jul 15:165(2): 1102-10) discloses insights on the biological role of CC chemokines in the Th1-mediated pathogenesis of spontaneous type I diabetes in nonobese diabetic (NOD) ice. It has been found that anti-MIP-1β treatment not only did not accelerate the development of diabetes in NOD.Scid recipients of transferred diabetogenic T cells but provided a small amount of protection of these recipients.

Kota et al. (Aberrant angiogenesis: The gateway to diabetic complications, Indian J. Endocrino.I Metab. 2012 Nov; 16(6):918-30) highlights that diabetes mellitus (DM) is a metabolic cum vascular syndrome of multiple etiology characterized by chronic hyperglycemia with disturbances of carbohydrate, fat, and protein metabolism, resulting from defects in insulin secretion, insulin action, or both leading to changes in both small blood vessels (microangiopathy) and large blood vessels (macroangiopathy), and that micro- and macrovascular beds are altered in diabetes by various changes in neovascular mechanism. It is further explained that excessive angiogenesis plays a role in diabetic retinopathy (DR), nephropathy, and in the vessel wall, potentially producing atherosclerotic plaque destabilization. Insufficient angiogenesis is stated to contribute to impaired wound healing and skin ulcers, impaired coronary collateral vessel (CV) development, embryonic vasculopathy in pregnancies complicated by maternal diabetes, and transplant rejection in diabetic recipients. Furthermore, diabetic neuropathy is described as a complication linked with reduced nutritive blood flow secondary to diabetes. It is detailed that defective arteriogenesis, a process of formation or remodeling of arterioles and arteries, has been reported in diabetic patients. Impaired release of endothelial progenitor cells (EPCs) from the bone marrow and defective function of these cells are identified as the other features of diabetes that further contribute to abnormal neovascularization and increased cardiovascular risk.

Meagher et al. (Neutralization of Interleukin-16 Protects Nonobese Diabetic Mice From Autoimmune Type 1 Diabetes by a CCL4-Dependent Mechanism, Diabetes, 2010 Nov; 59(11): 2862-2871) discloses that neutralization of CCL4 reverses anti-IL-16 mediated protection against type 1 diabetes, which is in agreement with several reports that CCL4 protects against T1D in mice and is not associated with disease onset in humans.

Meagher et al. (CCL4 protects from type 1 diabetes by altering islet beta-cell-targeted inflammatory responses, Diabetes, 2007 Mar; 56(3):809-17) discloses that antibody neutralization of CCL4 abrogates the ability of T-cells from IL-4-treated NOD mice to transfer protection against type 1 diabetes. Intradermal delivery of CCL4 via a plasmid vector stabilized by incorporation of the Epstein-Barr virus EBNA1/oriP episomal maintenance replicon (pHERO100-CCL4) to NOD mice beginning at later stages of disease progression was shown to protect against type 1 diabetes. This protection was associated with a Th2-like response in the spleen and pancreas; decreased recruitment of activated CD8+ T-cells to islets, accompanied by diminished CCR5 expression on CD8+ T-cells; and regulatory T-cell activity in the draining pancreatic lymph nodes. Thus, inflammatory responses that target islet β-cells were found to be suppressed by CCL4, implicating the use of CCL4 therapeutically to prevent type 1 diabetes.

Thus, we develop a new treatment strategy that increases the proliferation of endothelial progenitor cells, promotes angiogenesis and vessel repair, improves tissue ischemia in diabetic patients by inhibiting the function of macrophage inflammatory protein (MIP)-1β, and further to prevent and treat diabetic vasculopathy and cardiovascular diseases.

### SUMMARY OF INVENTION

The present invention has been found on the above purposes that direct inhibition of macrophage inflammatory protein-1β, such as through the monoclonal antibody and other methods, can produce effects of protecting blood the pancreas function, maintaining insulin secretion, and sopressing the continuous blood vessels in the diabetic animal models.

Accordingly, in one aspect, the present invention features a macrophage inflammatory protein-1 beta (MIP-1 beta) inhibitor for use in treating tissue ischemia in a diabetic subject, wherein the MIP-1 beta inhibitor is an antibody against MIP-1 beta.

### BREIF DESCRIPTION OF THE DRAWINGS

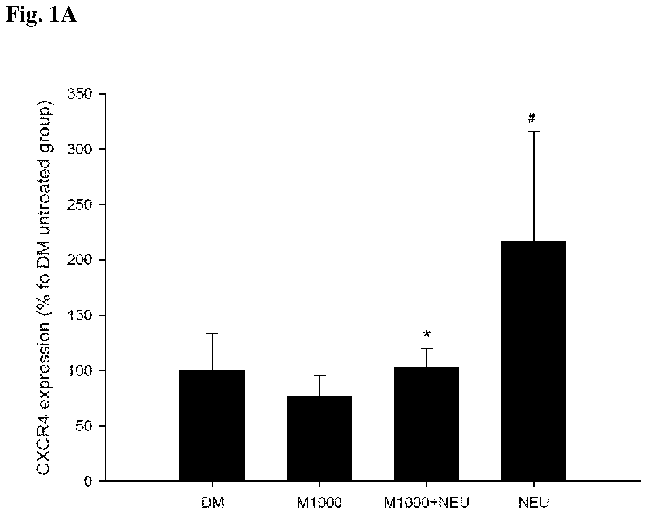

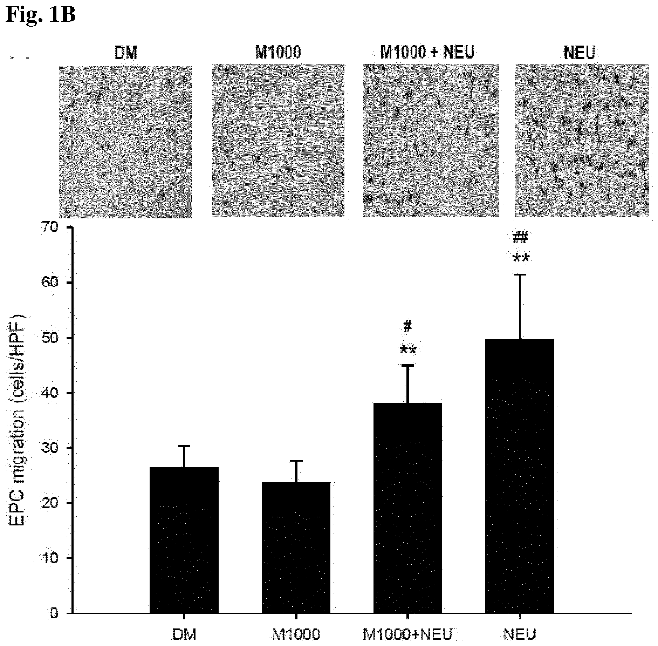

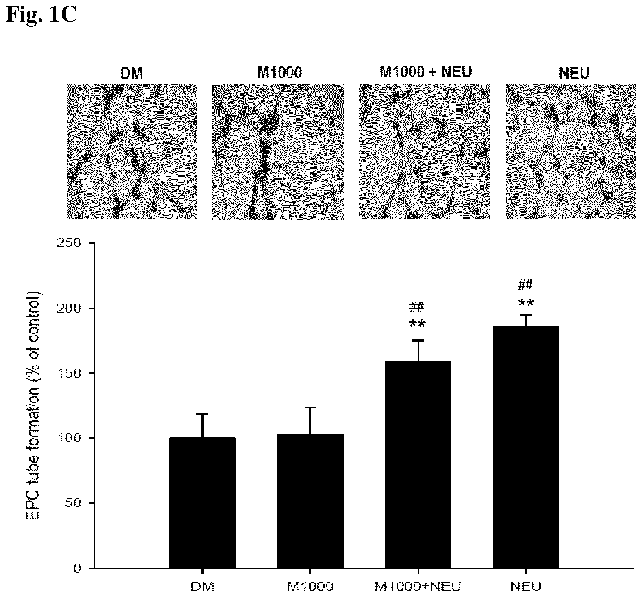

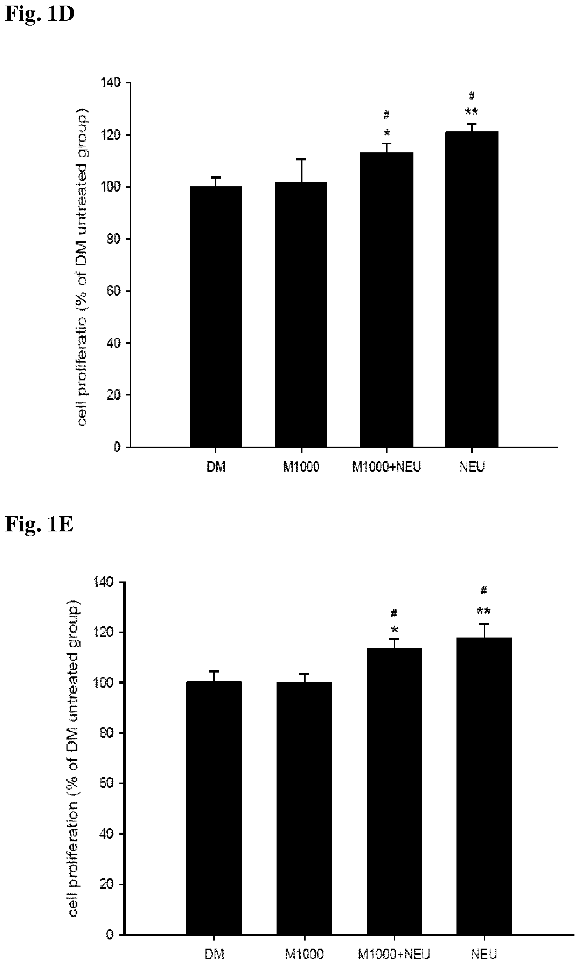

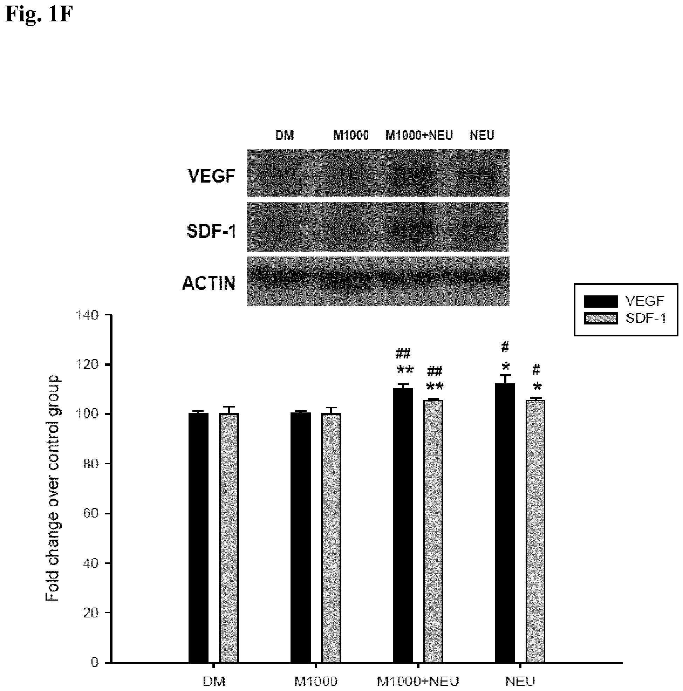

Fig. 1 shows MIP-1β inhibition sensitized CXCR4 expression and recovered functions of EPCs from diabetic patients and healthy subjects. CXCR4 expression on EPCs from diabetic patients and healthy subjects (1A; n=6). The migration and angiogenesis abilities in EPCs from diabetic patients (n=5; 1B, 1C). Cell proliferation was measured by MTT and BrdU cell proliferation assay in EPCs from diabetic patients (n=3; 1D, 1E). Western blot and satistical analysis of VEGF and SDF-1α expressions in EPCs from diabetic patients (n=3; 1F). #*P*<0.05, *##P<0.01* compared with untreated basal group. **P<0.05, **P<0.01* compared with MIP-1β 1000 ng/ml alone group.

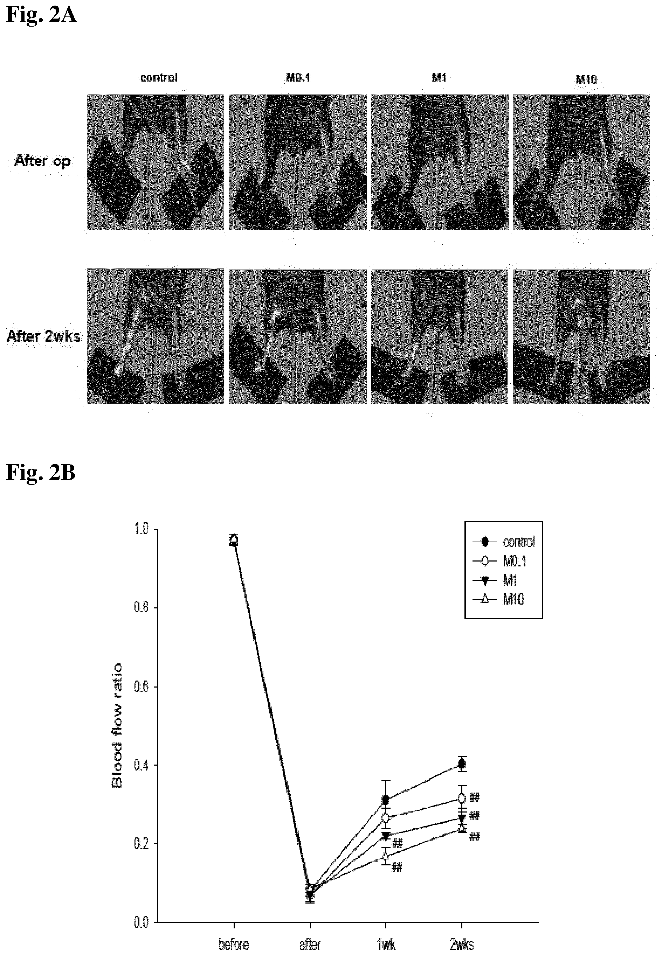

Fig. 2 shows the inhibitory effects of MIP-1β on neovasculogenesis in normal mice. Color-coded images and blood flow ratio (n=6~8; 2A, 2B).

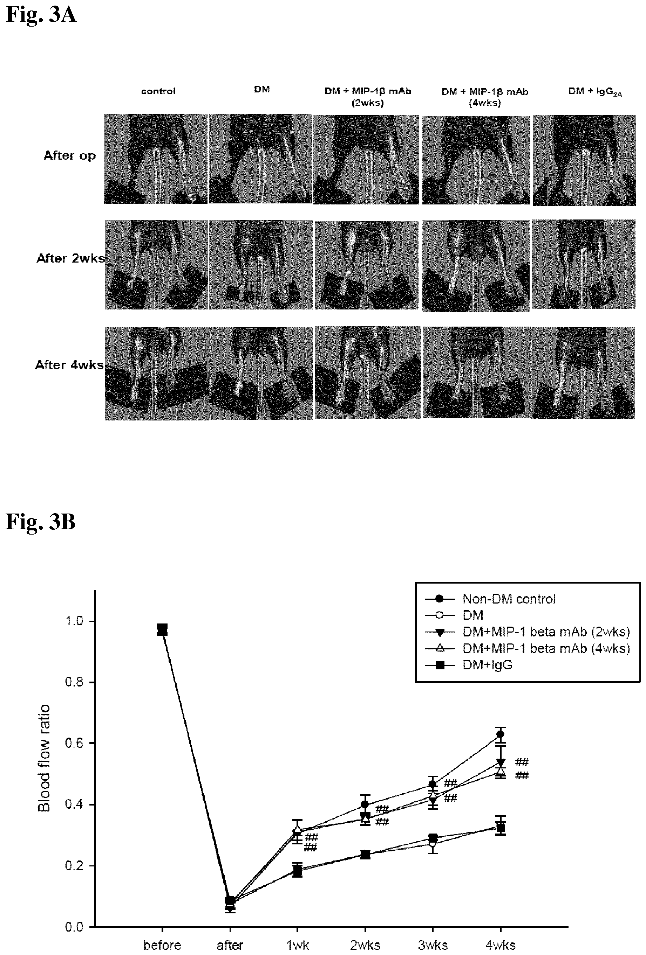

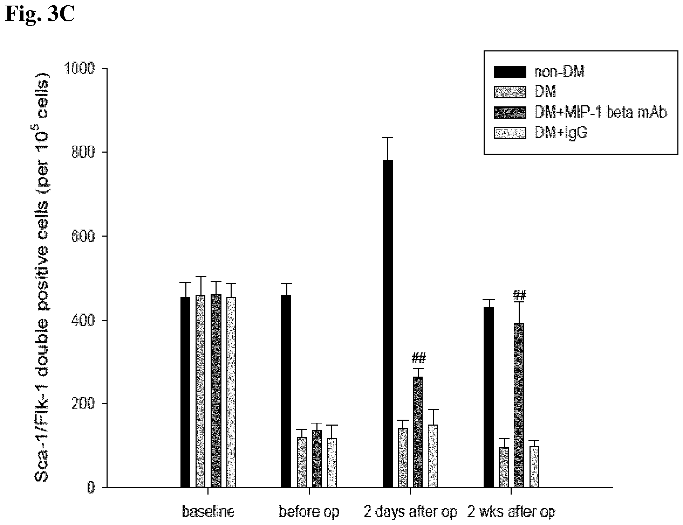

Fig. 3 shows the effects of MIP-1β inhibition on EPC homing in ischemia-induced neovasculogenesis in type 1 diabetic mice. Inhibition of MIP-1β activity by monoclonal antibody (mAb) will promote angiogenesis in diabetic mice with hindlimb ischemia operation (OP). Color-coded images and blood flow ratio (n=6~8; A, B). Circulating EPC and Western blot and satistical analysis of VEGF and SDF-1α after 2 weeks of MIP-1β neutralizing antibody injection in diabetic mice (n=6; C).

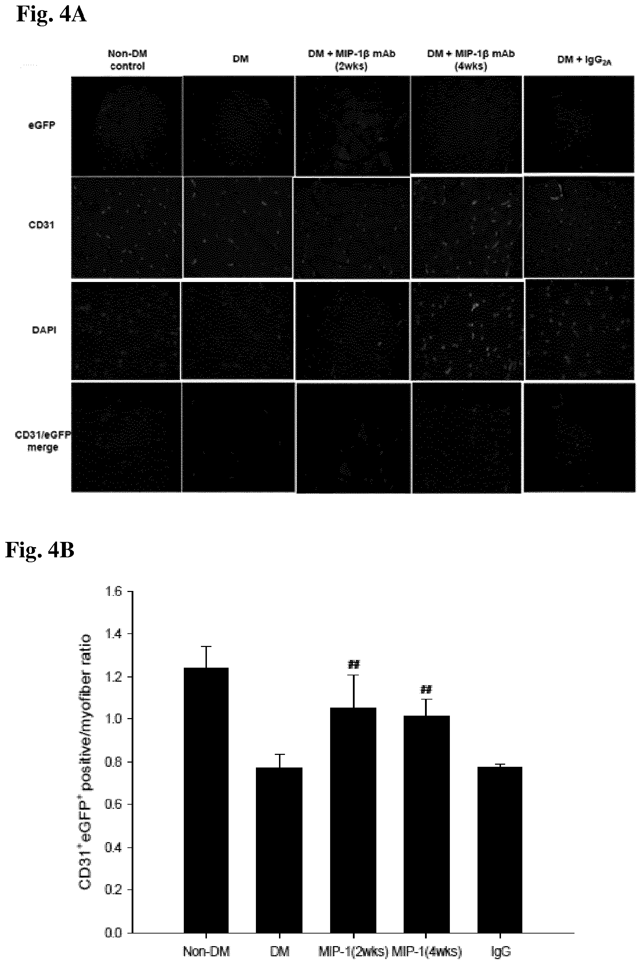

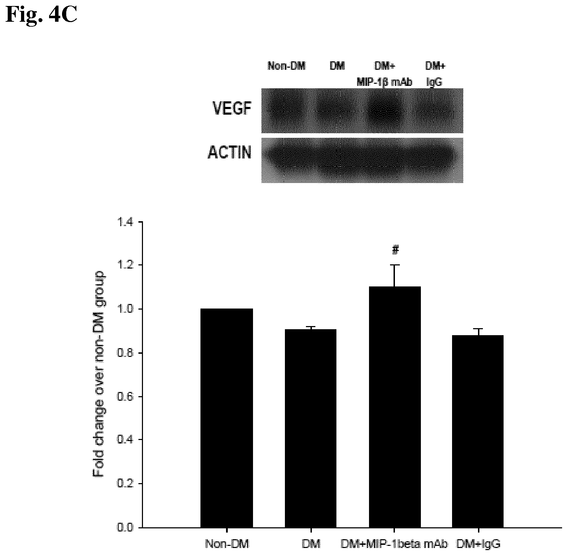

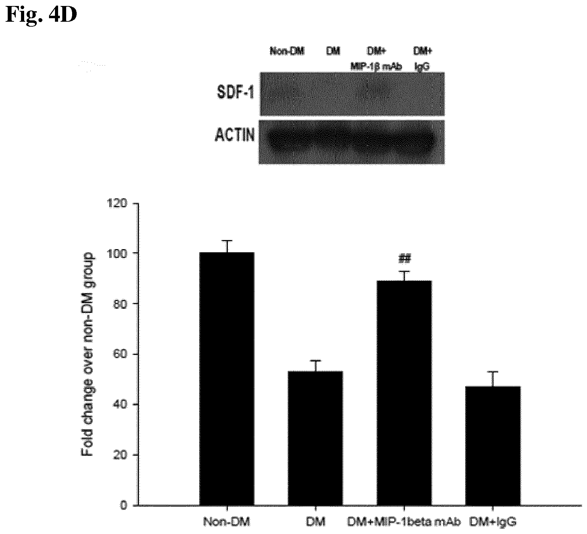

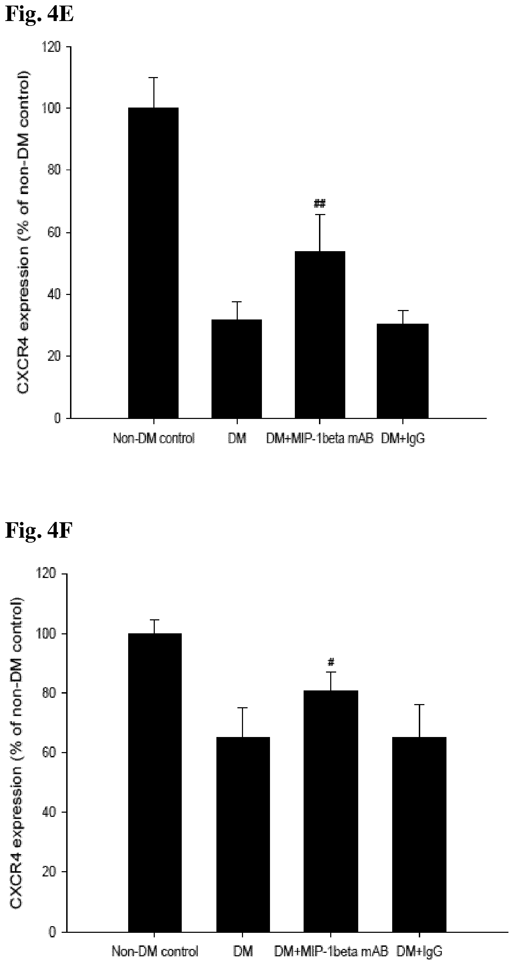

Fig. 4A shows the distribution and amount of the eGFP -CD31 (red fluorescence) double positive cells in the ischemic muscle analyzed by double staining immunohistochemical analysis. Figure 4B shows the signalling/myofibre ratio. (n=6~8). eGFP positive (with green fluorescence) cell represents bone marrow-derived endothelial progenitor cells. Capillaries were expressed in antibodies directed against CD31 (BD Pharmingen). EPC density was observed by eGFP/CD31 double-positive cells (yellow color) and evaluated by fluorescent microscopy. Fig. 4C and 4D show the Western blot and statistical analysis of VEGF and SDF-1α after 2 weeks of MIP-1β neutralizing antibody injection in diabetic mice. (n=3). Fig. 4E and 4F show the CXCR4 expression on peripheral mononuclear cells (E) and bone marrow mononuclear cells (F) (n=6~10). *#P<0.05, ##P<0.01* compared with untreated DM mice or control mice.

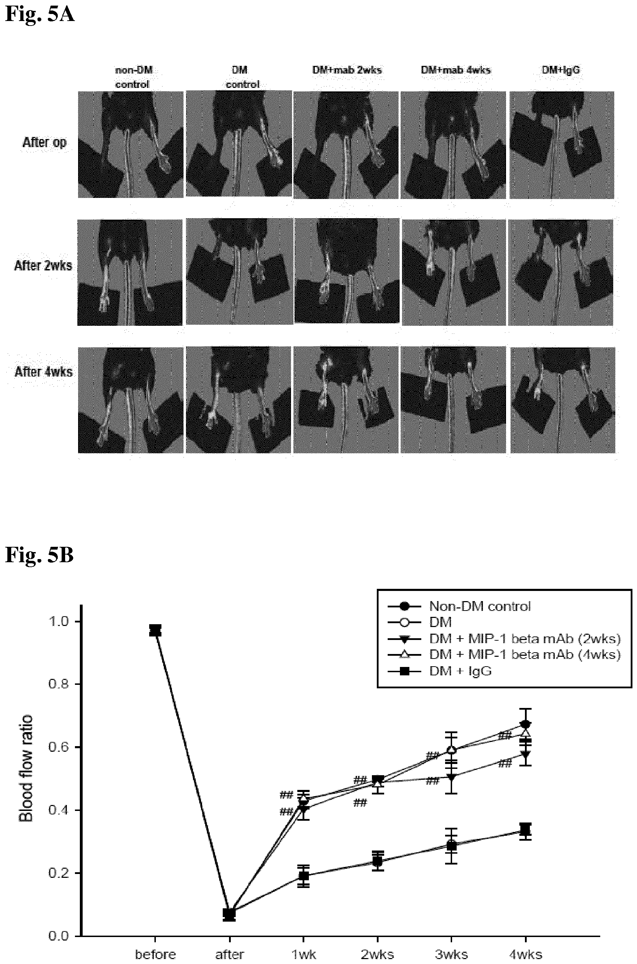

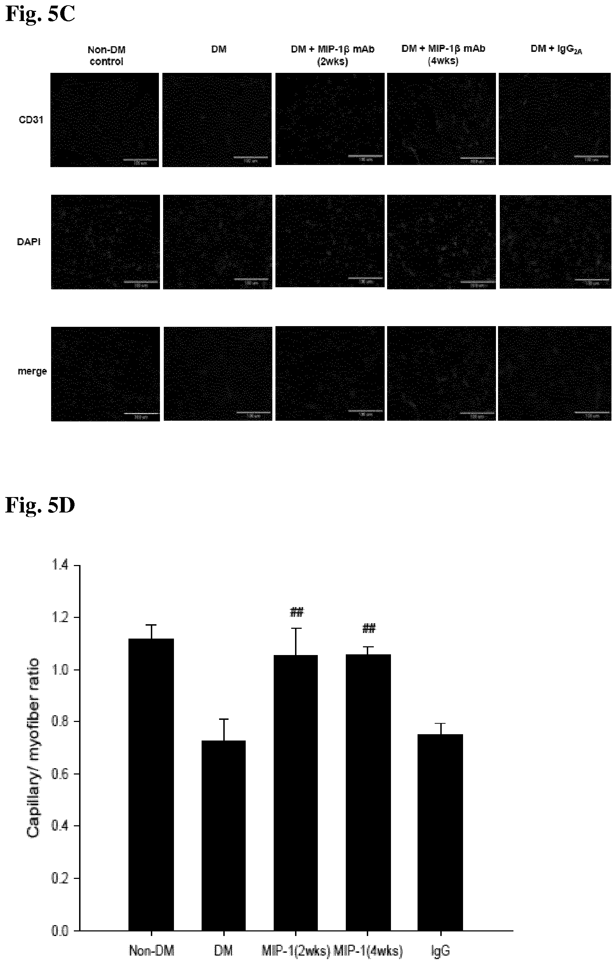

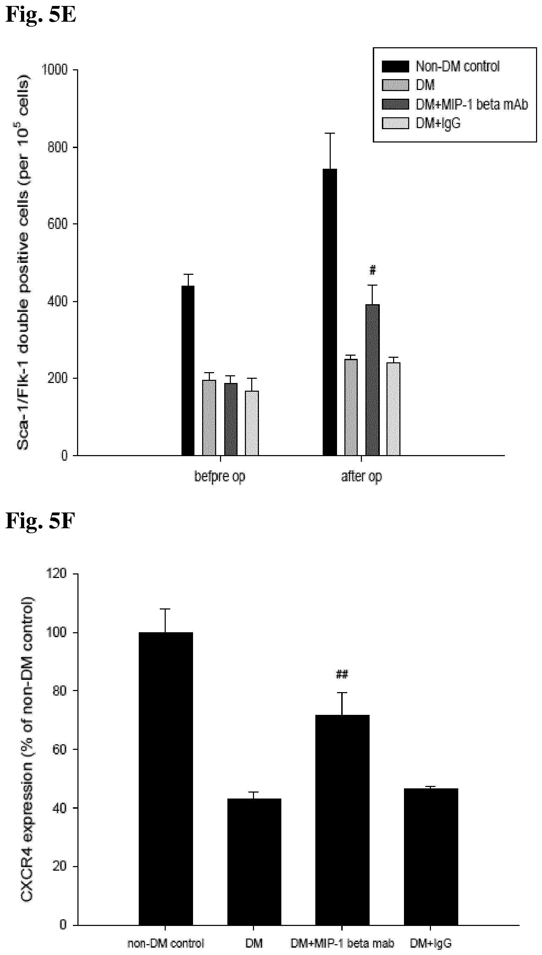

Fig. 5 shows the effects of MIP-1β neutralization for vessel protection in Lepr^{db/db} diabetic mice. Fig. 5A and 5B show that inhibition of MIP-1β activity by monoclonal antibody (mAb) will effectively promote angiogenesis in type 2 diabetic mice with hindlimb ischemia operation (OP). Color-coded images and blood flow ratio (n=6~8; A, B). MIP-1β inhibition increased capillary density in ischaemic muscle than untreated diabetic mice group. Fig. 5C and 5D are the histograms showing the increased capillary density (C) and the CD31 positive/myofibre ratio after the treatment of anti-MIP-1β monoclonal antibody (mAb) (n=6; C, D). Fig. 5E and 5F show the circulating EPC (n=6; E) and CXCR4 expression on bone marrow mononuclear cells (n=6; F) in the ischemic limb. *#P<0.05, ##P<0. 01* compared with untreated DM mice.

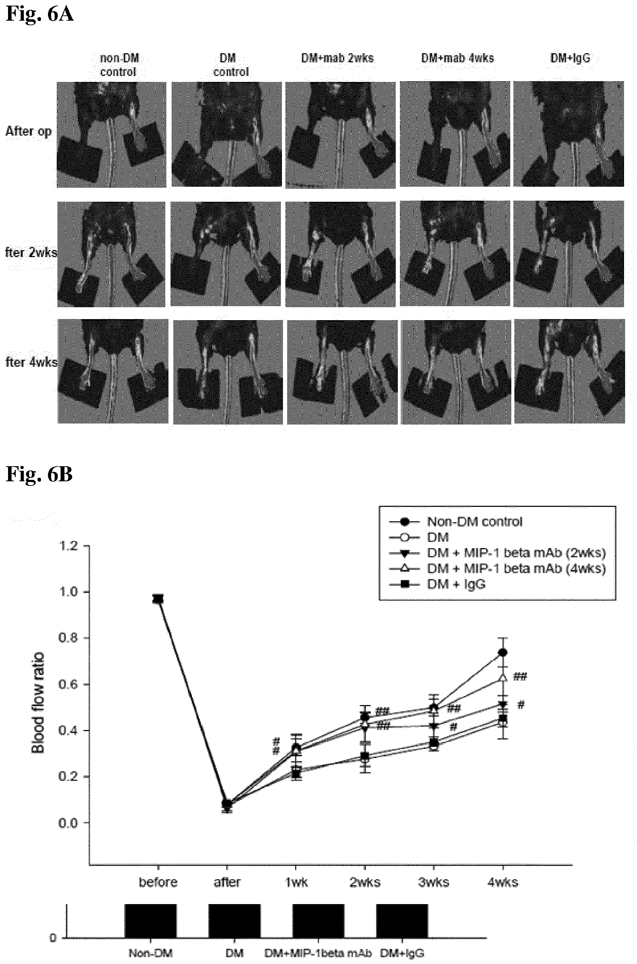

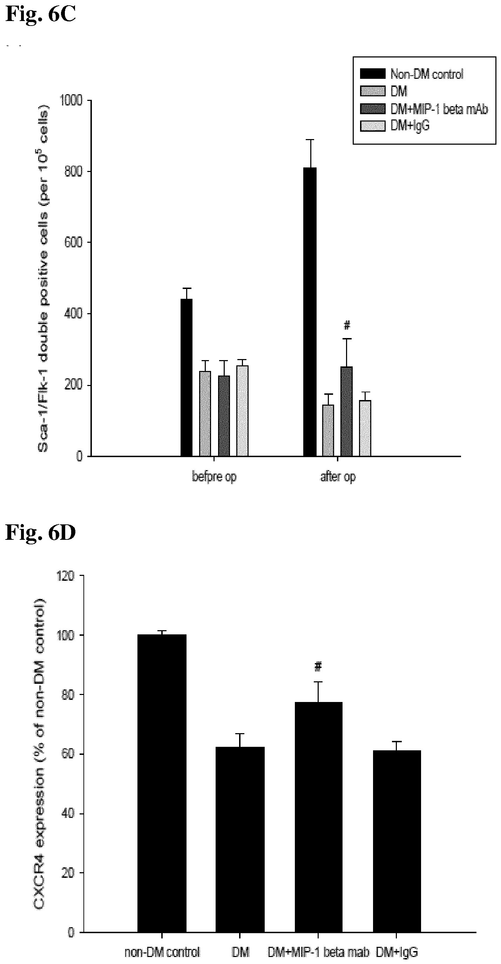

Fig. 6 shows the effects of MIP-1β neutralization for vessel protection, promoting ischemia-induced neovasculogenesis, in high fat diet-induced diabetic mice. Fig. 5A and 5B show the color-coded images (A) and blood flow ratio in the mAb treated diabetic mice after hindlimb ischemia operation (OP) (n=6). Fig. 6C and 6D show the circulating EPC (n=6; C) and CXCR4 expression on bone marrow mononuclear cells (n=6; D) in the ischemic limb. *#P<0.05, ##P<0.01* compared with untreated DM mice.

### DETAILED DESCRIPTION OF THE INVENTION

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

As used herein, "MIP-1β-inhibitor" refers to a compound that decreases the level of MIP-1β protein and/or decreases at least one activity of MIP-1β protein. In an exemplary embodiment, a MIP-1β-inhibiting compound may decrease at least one biological activity of a MIP-1β protein by at least about 10%, 25%, 50%, 75%, 100%, or more.

Methods for reducing, preventing or treating diseases or disorders using a MIP-1β-modulating compound may also comprise decreasing the protein level of a MIP-1β, or homologs thereof. Decreasing MIP-1β protein level can be achieved according to methods known in the art. For example, a siRNA, an antisense nucleic acid, or a ribozyme targeted to the MIP-1β can be expressed in or be transfected into the cell. Alternatively, agents that inhibit transcription can be used. Methods for modulating MIP-1β protein levels also include methods for modulating the transcription of genes encoding MIP-1β, methods for destabilizing the corresponding mRNAs, and other methods known in the art.

The MIP-1β-inhibitor directly or indirectly decreases or inhibits the activity of MIP-1β protein by binding to MIP-1β protein, and thereby to protect pancreas and prevent blood sugar from rising. For instance, methods for inhibiting the activity of MIP-1β protein in a subject may use an anti-MIP-1β antibody to compete with the MIP-1β protein for binding to its receptor on cell surface. The term "antibody" herein is used in the broadest sense and specifically includes full-length monoclonal antibodies, polyclonal antibodies, multispecific antibodies (e.g., bispecific antibodies), and antibody fragments, as long as they exhibit the desired biological activity.

As used herein, the term "antibody" means an immunoglobulin molecule or a fragment of an immunoglobulin molecule having the ability to specifically bind to a particular antigen. An "antibody fragment" comprises a portion of a full-length antibody, preferably antigen-binding or variable regions thereof. Examples of antibody fragments include Fab, Fab', F(ab)₂, F(ab')₂, F(ab)₃, Fv (typically the VL and VH domains of a single arm of an antibody), single-chain Fv (scFv), dsFv, Fd fragments (typically the VH and CH1 domain), and dAb (typically a VH domain) fragments; VH, VL, and VhH domains; minibodies, diabodies, triabodies, tetrabodies, and kappa bodies (see, e.g., Ill et al., Protein Eng 1997;10: 949-57); camel IgG; and multispecific antibody fragments formed from antibody fragments, and one or more isolated CDRs or a functional paratope, where isolated CDRs or antigen-binding residues or polypeptides can be associated or linked together so as to form a functional antibody fragment.

In certain embodiments, the MIP-1β-inhibitor is a monoclonal antibody specifically binding to the MIP-1β protein. In one embodiment, the anti-MIP-1β monoclonal antibody has the binding specificity for a functional fragment of MIP-1β protein structure. According to examples that do not form part of the invention but are useful for understanding the invention, the MIP-1β-inhibitor, such as a monoclonal antibody, binds to the antigen determinant fragment of MIP-1β protein comprising an amino acid sequence of ⁴⁶SFVMDYYET⁵⁴ (SEQ ID NO:1) or ⁶²AVVFLTKRGRQIC⁷⁴ (SEQ ID NO:2).

In some embodiments of the invention, the monoclonal antibody is a humanized antibody or a human antibody.

Pharmaceutical compositions for use in accordance with the present invention may be formulated in a conventional manner using one or more physiologically acceptable carriers comprising excipients and auxiliaries which facilitate processing of the active compounds into preparations which can be used pharmaceutically. Proper formulations, including (but not limited to) oral compositions such as tablets, capsules, powders and the like, parenteral compositions such as aqueous solutions for subcutaneous, intramuscular or intraperitoneal injection, and lyophilized powders combined with a physiological buffer solution just before administration, are formulated depending upon the chosen route of administration.

The other characteristics and advantages of the present invention will be further illustrated and described in the following examples. The examples described herein are using for illustrations, not for limitations of the invention.

### Example

### Example 1. MIP-1β inhibition sensitized CXCR4 expression and reversed functions of EPCs

To evaluate the direct effects of MIP-1β and MIP-1β inhibition on EPCs, MIP-1β 1000 ng/ml and MIP-1β antibody were alone or cotreated to EPCs (5x10⁴ cells) from diabetic patients for 30 min. As showed in **Fig. 1A****,** MIP-1β alone did not affect CXCR4 expression, and MIP-1β inhibition significantly increased the CXCR4 level on EPCs.

Thus, we examined if exposure of EPCs to MIP-1β could inhibit their migration toward SDF-1α by a chamber assay. As showed in **Fig. 1B** and **1C****,** the migration toward SDF-1α and *in vitro* neovasculogenesis abilities of MIP-1β treated EPCs (the M1000 group) were significantly inhibited. On the contrary, the migration and *in vitro* t neovasculogenesis were recovered in the MIP-1β inhibition groups (the M1000+NEU and NEU groups), as compared to the DM control and MIP-1β treatment (M1000) groups.

Cell proliferation was measured by MTT and BrdU cell proliferation assay in EPCs from diabetic patients. EPC proliferation was enhanced in the MIP-1β neutralizing antibody treated groups (**Fig. 1D, 1E**). In addition, MIP-1β inhibition could increase the level of VEGF and SDF-1α (**Fig. 1F**).

### Example 2. MIP-1β inhibition protects blood vessels in STZ-induced diabetic mice

In normal non-diabetic animals, the injection of MIP-1β could inhibit the vascular repair and angiogenesis after hindlimb ischemia operation (OP), reduce the restoration of blood flow in lower limbs, and enhance tissue ischemia (**Fig. 2**). These suppressive effects of MIP-1β on vessels are dose dependent as the increasing doses of MIP-1β (M0.1 µg, M1.0 µg, M10.0 µg).

Hindlimb ischemia was created in mice that induced to diabetic by STZ injection to confirm the effects of MIP-1β inhibition for enhancing neovasculogenesis in response to tissue ischemia in type 1 diabetic mice. After a 2-week stabilization period, hyperglycemia was generated in 6-week-old male FVB/NJNarl mice by the intraperitoneal injection of streptozotocin (40 mg/kg for 5 days).

The unilateral hindlimb ischemia was induced by excising the right femoral artery. Briefly, the proximal and distal portions of the right femoral artery and the distal portion of the right saphenous artery were ligated. The arteries and all side branches were dissected free and excised.

As showed in **Fig. 3A****,** blood flow in the ischemic hindlimb was equally reduced by hindlimb ischemia surgery in each group of mice. Perfusion recovery was markedly attenuated in the diabetic mice compared with non-diabetic ones during the postoperative weeks. The tissue ischemia was notable improved both in the MIP-1β antibody injection for 2 weeks or 4 weeks diabetic mice, and recovered to a similar blood flow ratio as compared with untreated diabetic ones (**Fig. 3B**)**.**

Furthermore, the increased number of Sca-1⁺/Flk-1⁺ EPC-like cell was attenuated in the untreated diabetic mice group compared with non-diabetic mice at 2 days after ischemic surgery. And, this attenuation of EPC-like cell number was recovered in the MIP-1β antibody injection group compared with untreated diabetic group. The number of EPC-like cells was still increased by MIP-1β inhibition for 2 weeks in diabetic mice (**Fig. 3C**).

### Example 3. MIP-1β inhibition improves neovasculogenesis by increasing EPC homing and upregulation of VEGF and SDF-1 in diabetic mice

To prove MIP-1β inhibition could improve bone marrow-derived EPC homing, the following *in vivo* experiments were tested by bone marrow transplantation model. Immunohistochemical analysis revealed that capillary density and homing number of bone marrow-derived EPC in the ischemic limb was reduced in diabetic mice compared with that in non-diabetic mice, and was increased in the MIP-1β antibody treated mice compared with that in untreated mice (**Fig. 4A, 4B**). The results suggest that MIP-1β inhibition can effectively increase the number of EPC cells (derived from bone marrow) and improve the reduction of capillary vessels in diabetic mice due to tissue ischemia. eGFP positive (green fluorescence) cells represented bone marrow-derived EPCs. Capillaries were expressed in antibodies directed against CD31 (BD Pharmingen) as showed in red color. DAPI staining (blue fluorescence) represents the nucleus EPC density was observed by eGFP/CD31 double-positive cells and evaluated by fluorescent microscopy, showing the cells with capacity of moving to the ischemic position and repairing blood vessel. For the standardized quantitation, the overlapped amount of green and red fluorescence was presented as a ratio with the number of muscle fibers.

And, the expressions of VEGF and SDF-1α in the ischemic muscle were decreased in diabetic mice compared with non-diabetic mice. MIP-1β inhibition group had higher amounts of VEGF and SDF-1α in the ischemic muscle than untreated diabetic ones **(****Figure 4C****,** **4D**). MIP-1β inhibition also reversed the decreased CXCR4 expression on peripheral and bone marrow mononuclear cells in diabetic mice (**Fig. 4E, 4F**).

These findings proved that MIP-1β inhibition could enhance neovasculogenesis in response to tissue ischemia in diabetic mice through increasing EPC-like cell homing, enhancement of VEGF and SDF-1α, and reversed the expression of CXCR4 on peripheral and bone marrow mononuclear cells, which will restore normal blood flow and prevent necrosis in damaged tissue.

### Example 4. MIP-1β inhibition protects blood vessels and enhances ischemia-induced neovasculogenesis in Lepr^{db/db} type 2 diabetic mice

The effects of MIP-1β inhibition in enhancing neovasculogenesis and recovering blood flow of ischemic limbs in type 2 diabetic animals were proved by in vivo tests in Lepr^{db/db} diabetic mice. As the results showed in **Fig. 5****,** MIP-1β neutralization by mAb injection could effectively assist neovasculogenesis after the hindlimb ischemia surgery (OP), increase the recovery of blood flow in hindlimb, and improve tissue ischemia in type 2 diabetic mice (**Fig. 5A, 5B**).

Similarly, as the results observed in type 2 diabetic mouse model, MIP-1β inhibition enhanced the circulating EPC number in peripheral vessels (**Fig. 5C**), increased the density of capillary (**Fig. 5D**), and recovered the bone marrow-derived EPC homing number (**Fig. 5E**), and restored CXCR4 expression on bone marrow mononuclear cells (**Fig. 5F**) in diabetic animals with hindlimb ischemia surgery (OP).

### Example 5. MIP-1β inhibition protects blood vessels and enhances ischemia-induced neovasculogenesis in high fat diet-induced diabetic mice

In this example, the effects of MIP-1β inhibition in enhancing neovasculogenesis and recovering blood flow of ischemic limbs in obese diabetic animals were also evaluated in the high fat diet-induced diabetic mouse model. As t showed in **Fig. 6****,** MIP-1β neutralization by mAb injection could effectively assist neovasculogenesis after the hindlimb ischemia surgery (OP), increase the recovery of blood flow in hindlimb, and improve tissue ischemia in the type 2 diabetic mice with high blood sugar level (**Fig. 6A, 6B**).

In the obese type diabetic mouse model, MIP-1β inhibition could effectively increase the Sca-1+/Flk-1+ EPC-like cell number in peripheral vessels (**Fig. 6C**), and restored CXCR4 expression on bone marrow mononuclear cells (**Fig. 6D**) in diabetic animals after the hindlimb ischemia surgery (OP).

These findings showed in the present invention have proved that MIP-1β could suppress neovasculogenesis, and reduce the recovery of blood flow after limb ischemic, aggravating tissue ischemia in normal and diabetic mice. As one embodiment of inhibitory agent for MIP-1β, the MIP-1β neutralizing antibody exhibits effects on diabetic animals, including protecting vessels through increasing EPC-like cell proliferation and homing, enhancing neovasculogenesis, and improving tissue ischemia. Therefore, It has a direct and significant benefit to the prevention and treatment of diabetic vasculopathy by directly inhibiting the *in vivo* activity and action of macrophage inflammatory protein (MIP)-1β (for example, by administering monoclonal antibody or antagonist against MIP-1β.

## Claims

1. A macrophage inflammatory protein-1 beta (MIP-1 beta) inhibitor for use in treating tissue ischemia in a diabetic subject, wherein the MIP-1 beta inhibitor is an antibody against MIP-1 beta.

## Patentansprüche

1. Makrophagen-Entzündungsprotein-1 beta (MIP-1 beta) Inhibitor zur Verwendung bei der Behandlung von Gewebeischämie bei einem diabetischen Subjekt, wobei der MIP-1 beta Inhibitor ein Antikörper gegen MIP-1 beta ist.

## Revendications

1. Inhibiteur de la protéine inflammatoire de macrophages-1 bêta (MIP-1 bêta) utilisé pour traiter l'ischémie tissulaire chez un sujet diabétique, dans lequel l'inhibiteur de MIP-1 bêta est un anticorps dirigé contre MIP-1 bêta.
